# EUROPÄISCHE PATENTANMELDUNG

(11) **EP 2 907 510 A1**
(43) Veröffentlichungstag der Anmeldung: **19.08.2015**
(21) Anmeldenummer: 15000734.2
(22) Anmeldetag: 22.05.2007
(51) Int. Cl.: A61K 9/70, A61K 31/485

(54) **Selbstzerstörendes transdermales therapeutisches System**

(30) Priorität: 31.05.2006 DE 102006025282
(62) Teilanmeldung aus: 07764544.8
(71) Anmelder: LTS Lohmann Therapie-Systeme AG, 56626 Andernach (DE)
(72) Erfinder: Hoffmann, Hans-Rainer, D-56566 Neuwied (DE); Hille, Thomas, D-56567 Neuwied (DE)
(74) Vertreter: Schweitzer, Klaus

(57) **Zusammenfassung**

Die Erfindung betrifft ein transdermales therapeutisches System (TTS), bevorzugt in Form eines transdermalen Pflasters, welches einen Wirkstoff, ein den Wirkstoff unbrauchbar machendes Agens und ein Mittel enthält, das bewirkt, dass beim Abnehmen des TTS von der Haut des Patienten Wirkstoff, z. B. Buprenorphin, und Agens, z. B. Kaliumpermanganat, miteinander in Berührung kommen und durch diesen Kontakt der Wirkstoff zerstört wird.

## Beschreibung

Die Erfindung betrifft ein sich nach Gebrauch selbstzerstörendes, transdermales therapeutisches System oder auch transdermales Pflaster genannt (TTS). Das erfindungsgemäße TTS enthält einen therapeutischen Wirkstoff, bevorzugt aus der Gruppe der Schmerzmittel.

So sind z.B. TTS mit den Wirkstoffen Buprenorphin und Fentanyl die Arzneiformen der Wahl zur Behandlung von chronischen Schmerzen in der Langzeittherapie. Durch die kontinuierliche Abgabe von diesen hochwirksamen Schmerzmitteln über die Haut wird ein Schmerzpatient kontinuierlich mit einem Schmerzmittel versorgt, so dass Plasmaspitzen und Plasmatäler vermieden werden.

Dies hat den Vorteil, dass durch eine niedrige aber ausreichende Plasmakonzentration des Wirkstoffs sowohl Nebenwirkungen durch Überdosierungen, aber auch Schmerzzustände durch Unterversorgung, vermieden werden. Dem Fachmann sind z. B. die Handelsprodukte Transtec^{®}, aber auch Durogesic^{®} oder Durogesic Smat bekannt, die sich schon seit längerer Zeit in der Schmerztherapie bewährt haben. Der Nachteil der TTS in der Schmerztherapie besteht darin, dass zur Aufrechterhaltung des so genannten Konzentrationsgradienten und damit des gewünschten Plasmaspiegels des Wirkstoffs während der Applikationsdauer der TTS immer mehr Wirkstoff im TTS enthalten sein muss als tatsächlich an den Patienten abgegeben wird. Dies hat zur Folge, dass getragene TTS ein Missbrauchspotential für z. B. Angehörige der Drogenszene darstellen, da diese Personengruppen durchaus in der Lage sind, benutzte TTS zu sammeln und mit primitivsten Hilfsmitteln zu extrahieren, um den noch enthaltenen Wirkstoff zu gewinnen und zur Befriedigung der Drogensucht zu missbrauchen.

Es hat daher in der Vergangenheit nicht an Versuchen gemangelt, diesen Missbrauch dadurch zu unterbinden, dass Patienten geraten worden ist, das getragene Pflaster zu zerschneiden und durch die Toilette der Kanalisation zuzuführen. Nachteilig an diesem Verfahren ist, dass weder Gesetzgeber noch Arzneimittelhersteller die Gewähr übernehmen können, dass diese Maßnahme von den Patienten auch tatsächlich befolgt wird. Daher wurden TTS entwickelt, die neben dem Wirkstoff auch einen Antagonisten enthielten (z. B. WO 2004/098576, WO 90/04965, WO 2004/037259). Dadurch sollte die oben beschriebene Gewinnung bzw. Extraktion des schmerzstillenden Wirkstoffs aus gebrauchten TTS verhindert zumindest aber deutlich erschwert werden. Diese Schutzmaßnahmen haben sich jedoch als nicht ausreichend zur Vermeidung von Medikamentenmissbrauch erwiesen, da nach wie vor mit relativ einfachen Mitteln der eigentliche Wirkstoff durch fraktionierte Fällung von dem Antagonisten getrennt werden kann.

In der WO 02/094172 ist ein System zur Missbrauchsvermeidung von Dosierungssystemen beschrieben, jedoch bleibt in diesem System der Wirkstoff nach wie vor aktivierbar und wird nicht zerstört. Ebenso in der WO 2005/070003; dort wird der Wirkstoff lediglich absorbiert, welches nach wie vor die Möglichkeit der Abtrennung von dem Ab-/Adsorbens ermöglicht. Schließlich wird auch in der WO 2004/098568 ein ,Missbrauchsresistentes' transdermales Dosierungssystem beschrieben. Ebenso wie in den übrigen bekannten Systemen dieser Art wird auch hier der Wirkstoff nicht zerstört, sondern lediglich durch einen Antagonisten in der Wirkung neutralisiert.

Der vorliegenden Erfindung lag daher die Aufgabe zu Grunde, ein TTS bereitzustellen, bei dem nach Gebrauch der beschriebene Medikamentenmissbrauch zumindest weitgehend ausgeschlossen werden kann.

Gelöst wird diese Aufgabe durch die Bereitstellung eines TTS, bevorzugt in Form eines auf die Hautoberfläche des Patienten aufzubringenden transdermalen Pflasters, dass sich nach Gebrauch, d. h. nach Abnahme des TTS von der Hautoberfläche des Patienten, - automatisch - selbst zerstört. Sich selbst zerstörendes TTS bedeutet primär, dass der enthaltene Arzneimittelwirkstoff nach Gebrauch zerstört, chemisch umgesetzt und/oder unbrauchbar gemacht wird. Dabei ist gewährleistet, dass dieser Zerstörungsprozess nicht vor oder während der Applikation des TTS gestartet wird.

Gegenstand der Erfindung ist somit ein transdermales therapeutisches System (TTS), bevorzugt in Form eines transdermalen Pflasters, welches zumindest einen therapeutischen Wirkstoff und eine Substanz oder ein Substanzgemisch (Agens) enthält, welches den Wirkstoff, bevorzugt durch chemische Reaktion, zerstören bzw. unbrauchbar machen kann, wobei Wirkstoff und Agens voneinander getrennt (bevorzugt räumlich getrennt) vorliegen und wobei das TTS zumindest ein Mittel enthält, das bewirkt, dass beim Abnehmen des TTS von der Haut des Patienten Wirkstoff und Agens miteinander in Berührung kommen und der Wirkstoff durch diesen Kontakt zerstört oder in seiner Wirksamkeit unbrauchbar gemacht wird.

Bei dem Agens kann es sich um eine Substanz oder ein Substanzgemisch handeln, die wiederum als Feststoff, Lösung, Gel, Dispersion oder anderen Erscheinungsformen vorliegen können. Bevorzugt ist das Agens eine Substanz, die mit dem Wirkstoff chemisch reagiert und ihn dadurch zerstört, insbesondere ein chemisches Oxidationsmittel wie z. B. anorganische Reagenzien, wie Permanganate, z.B. Kaliumpermanganat, Mangandioxid, Bleidioxid, Bleitetraacetat, Cer(IV)-Salze, Chromate, Chromsäure, Osmiumtetroxid, Salpetersäure, Nitrite, wie Kaliumnitrit, Selendioxid, Wasserstoffperoxid und andere Peroxo-Verbindungen, Brom, Chlor, Hypohalogenide, oder Schwefel; bevorzugt Kaliumpermanganat, Wasserstoffperoxid und Kaliumnitrit; organische Oxidantien, wie Dimethylsulfoxid, N-Bromsuccinimid, Chinone, hypervalente lodverbindungen, Persäuren und Perester, aber auch Enzyme. Bei gegebenem Wirkstoff wird das Agens bevorzugt aufgrund seiner chemischen Reaktionsfähigkeit mit dem Wirkstoff ausgewählt.

Bei dem Wirkstoff handelt es sich bevorzugt um einen Wirkstoff aus der Gruppe der Schmerzmittel wie z. B. Narkotika. Bevorzugt sind zu nennen Morphinderivate, Heroin und Buprenorphin, oder Fentanyl und seine Derivate Sufentanyl und Alfentanyl. Grundsätzlich sind auch alle anderen Wirkstoff/Agens - Kombinationen verwendbar, für die die Applikation über ein TTS die geeignete Darreichungsform ist. Das Mittel, das bewirkt, dass beim Abnehmen des Pflasters / TTS von der Haut der Patienten Wirkstoff und Agens miteinander in Berührung und/oder zur chemischen Reaktion miteinander gelangen, kann ebenfalls in vielfältigen Formen auftreten. Es muss gewährleistet sein, dass bei jeder Abnahme des TTS, unabhängig von der Abzugsrichtung, das Mittel seine Funktion erfüllt. Das Mittel wird dabei auf die Form abgestimmt, in der das Agens vorliegt (z. B. als Lösung in einem Beutel). Bevorzugt ist das Mittel innen an der äußeren Deckschicht des TTS fixiert, z. B. durch verkleben mit der Innenseite der Deckschicht. Beispiele für erfindungsgemäße Mittel in Abhängigkeit von der Erscheinungsform des Agens erschließen sich dem Fachmann mit der Auswahl des Agens und seiner Unterbringungsform im TTS.

Ansonsten können für die Herstellung des erfindungsgemäßen TTS bzw. transdermalen Pflasters die Materialien verwendet werden, die dem Fachmann für solche Systeme bekannt sind.

Das erfindungsgemäße TTS hat bevorzugt einen Schichtaufbau, wofür eine mögliche Variante im Ausführungsbeispiel exemplarisch erläutert wird. Das TTS kann in Form eines Matrix-Pflasters vorliegen, bei dem der Wirkstoff in einer aus einer oder mehreren Schichten bestehenden Matrix enthalten ist, die mit Hilfe einer Kleberschicht direkt auf der Haut aufliegt. Bei der ebenfalls möglichen Ausbildung als Membranpflaster liegt zwischen dem Wirkstoff-Reservoir und der Haut eine klebende Membran, welche die Wirkstoffabgabe in die oberste Schicht der Haut, die Epidermis, steuert.

Für die Herstellung des erfindungsgemäßen TTS kann der Fachmann somit grundsätzlich auf die Materialien, Herstellungsverfahren und den Aufbau der aus dem Stand der Technik bekannten TTS bzw. transdermalen Pflaster zurückgreifen, die erfindungsgemäß zusätzlich eine geeignete Mittel / Agens - Kombination aufweisen (vgl. z. B. Transdermale Pflaster; Spektrum der Wissenschaft 10/2003, 42; Transdermal Controlled Systemic Madications, Y.W. Chien, Drugs and the Pharmaceutical Sciences, Vol. 31; Polymers in Transdermal Drug Delivery Systems, S. Kandavilli et. al., Pharmaceutical Technology, May 2002, 62-80). Voraussetzung für die Eignung eines Kunststoffes für solche medizinischen Anwendungen ist neben günstigen Werkstoffeigenschaften (z.B. mechanische Festigkeit und Verarbeitbarkeit), aus hygienischen Gründen insbesondere dessen gute Sterilisierbarkeit. Diese Anforderungen werden z.B. von Polyethylen, Polypropylen, Polyvinylchlorid, Polystyrol, Polymethacrylaten, Polyamiden und Polycarbonaten erfüllt.

Die Erfindung wird durch das folgende Beispiel näher erläutert. Gleichwohl können spezielle, in dem Beispiel beschriebene Ausgestaltungen des erfindungsgemäßen TTS einzeln oder in Kombination miteinander als bevorzugte Merkmale für die Erfindung als solche verallgemeinert werden.

### Beispiel 1

Zu 1,14 kg einer Lösung eines selbst vernetzenden Polyacrylates, bestehend aus den Monomeren 2-Ethylhexylacrylat, Vinylacetat, Butylacrylat und Acrylsäure in der Mischung der organischen Lösemittel Ethylacetat, Heptan und Isopropanol/Toluol, gibt man 100 g Lävulinsäure, 150 g Oleyloleat, 100 g Polyvinylpyrrolidon, 150 g Ethanol, 200 g Ethylacetat und 100 g Buprenorphin Base und rührt diese Mischung bis zur Homogenisierung für etwa 2 Stunden. Nach dem Homogenisieren streicht man die Mischung auf die silikonisierte Seite einer 100 µm Polyesterfolie und entfernt die Lösemittel im Trockenschrank durch 10minütiges Trocknen bei 60 bzw. 80°C. Die Strichstärke in der Beschichtung wurde so gewählt, dass nach Entfernen des Lösemittels ein Flächengewicht von ca. 80 g/m² resultiert. Nach Entfernen der Lösemittel wird das Laminat bestehend aus silikonisierter Polyesterfolie und wirkstoffhaltiger Polymerschicht mit einem saugfähigen Material, z. B. Löschpapier oder einem Vlies abgedeckt. Danach zerschneidet man das gesamte Laminat in Quadrate der Kantenlänge 5 x 5 cm. Man entfernt die 5 x 5 cm große silikonisierte Polyesterfolie und legt das Laminat aus buprenorphinhaltiger Kleberschicht und Vlies auf die silikonisierte Seite einer weiteren Polyesterfolie in einer Art und Weise auf, dass die Polyesterfolie die wirkstoffhaltige Kleberschicht, abgedeckt mit saugfähigem Hartvlies, allseitig überragt. Nun legt man auf das Vlies einen fünfzackigen Stern aus hartem Plastikmaterial auf. Auf das saugfähige Vlies legt man einen Beutel, gefüllt mit Kaliumpermanganatlösung auf, der so gestaltet ist, dass er in seiner Gesamtfläche kleiner ist als die wirkstoffhaltige Polymerschicht. Ohne die Erfindung einzuschränken, kann der Beutel Maße von 4 x 4 cm aufweisen. In einem zweiten Arbeitsschritt hat man zuvor ein Laminat, bestehend aus silikonisiertem Papier, wirkstofffreier Haftkleberschicht und Polyesterfolie 23 µm, hergestellt. Man entfernt das silikonisierte Papier und deckt das Zwischenprodukt, bestehend aus silikonisierter Polyesterfolie, dem Quadrat, bestehend aus wirkstoffhaltiger Polymerschicht mit saugfähigem Vlies und Stern, abgedeckt von einem 4 x 4 cm großen Polyethylenbeutel, gefüllt mit Kaliumpermanganatlösung, ab und stanzt nun die TTS in einer Art und Weise aus, dass die wirkstofffreie Haftkleberschicht die wirkstoffhaltige Haftkleberschicht allseitig überragt.

Wird nun das TTS appliziert, muss man zunächst einmal die silikonisierte Polyesterschicht (Releaseliner) entfernen, was leicht möglich ist. Wird das TTS auf die Haut eines Patienten aufgeklebt, so bleibt der Beutel, gefüllt mit wässriger Kaliumpermanganatlösung, unbeschädigt. Wird aber nach der Applikationsdauer von 2 - 7 Tagen das TTS von der Haut des Patienten entfernt, so bohrt sich mindestens eine Zacke des fünfzackigen Sterns aufgrund der Steifigkeit durch den Beutel mit Kaliumpermagnatlösung und zerstört diesen zwangsläufig. Durch die Geometrie des Sterns ist sichergestellt, dass der Beutel in jedem Falle reißt, egal in welcher Richtung das TTS vom Patienten entfernt wird. Durch das saugfähige Vlies verteilt sich die Kaliumpermanganatlösung innerhalb einer kurzen Zeit über die Fläche des TTS. Dadurch wird ein Oxydationsprozess gestartet, der im Falle von z.B. Buprenorphin zu dessen oxydativer Zerstörung führt. Selbst wenn das getragene TTS unmittelbar nach TTS-Entfernung der Extraktion zugeführt wird, ist dieser Zersetzungsprozess nicht mehr aufzuhalten, im Gegenteil, er beschleunigt sich dadurch, dass sowohl das Opiat Buprenorphin als auch das Oxydationsmittel Kaliumpermanganat in Lösung gebracht werden. Dadurch ist sichergestellt, dass der Wirkstoff nicht missbraucht werden kann.

Das im Beispiel beschriebene transdermale Pflaster hat somit folgenden (Schicht-) Aufbau (1 - 6):
- 6: Polyesterfolie mit wirkstofffreier Haftkleberschicht
- 5: Plastikstern
- 4: Kaliumpermanganatlösung (Beutel)
- 3: Vlies
- 2: wirkstoffhaltige Haftkleberschicht
- 1: silikonisierte Polyesterschicht (Releaseliner)
- 0: Haut

## Patentansprüche

1. Transdermales therapeutisches System (TTS) enthaltend einen Wirkstoff, ein den Wirkstoff unbrauchbar machendes Agens und ein Mittel, das bewirkt, dass beim Abnehmen des TTS von der Haut des Patienten Wirkstoff und unbrauchbar machendes Agens miteinander in Berührung kommen und durch diesen Kontakt der Wirkstoff zerstört wird.

2. Transdermales therapeutisches System nach Anspruch 1, **dadurch gekennzeichnet, dass** das TTS ein transdermales Pflaster ist.

3. Transdermales therapeutisches System nach Anspruch 1 oder 2, **dadurch gekennzeichnet, dass** Wirkstoff und Agens räumlich voneinander getrennt sind.

4. Transdermales therapeutisches System nach einem oder mehreren der Ansprüche 1 bis 3, **dadurch gekennzeichnet, dass** Wirkstoff und Agens nach dem Abnehmen des TTS von der Haut des Patienten chemisch miteinander reagieren.

5. Transdermales therapeutisches System nach einem oder mehreren der Ansprüche 1 bis 4, **dadurch gekennzeichnet, dass** der Wirkstoff ein Schmerzmittel ist.

6. Transdermales therapeutisches System nach einem oder mehreren der Ansprüche 1 bis 5, **dadurch gekennzeichnet, dass** der Wirkstoff ein Narkotikum ist.

7. Transdermales therapeutisches System nach einem oder mehreren der Ansprüche 1 bis 6, **dadurch gekennzeichnet, dass** der Wirkstoff ein Morphinderivat, Heroin oder Buprenorphin ist.

8. Transdermales therapeutisches System nach einem oder mehreren der Ansprüche 1 bis 7, **dadurch gekennzeichnet, dass** der Wirkstoff Fentanyl, Sufentanyl oder Alfentanyl ist.

9. Transdermales therapeutisches System nach einem oder mehreren der Ansprüche 1 bis 8, **dadurch gekennzeichnet, dass** das Agens ein Oxidationsmittel ist, bevorzugt Kaliumpermanganat, oder dass es ein Enzym ist.

10. Transdermales therapeutisches System nach einem oder mehreren der Ansprüche 1 bis 9, **dadurch gekennzeichnet, dass** es eine äußere Deckschicht umfaßt und dass das Mittel, das bewirkt, dass beim Abnehmen des TTS von der Haut des Patienten Wirkstoff und unbrauchbar machendes Agens miteinander in Berührung kommen, an der Innenseite der äußeren Deckschicht fixiert ist, wobei es bevorzugt mit der Innenseite verklebt ist.

11. Transdermales therapeutisches System nach einem oder mehreren der Ansprüche 1 bis 10, **dadurch gekennzeichnet, dass** der Wirkstoff Buprenorphin und das Agens Kaliumpermanganat ist.

12. Transdermales therapeutisches System nach einem oder mehreren der Ansprüche 1 bis 11, **dadurch gekennzeichnet, dass** es als Membranpflaster ausgebildet ist, bei dem zwischen dem Wirkstoff-Reservoir und der Haut eine klebende Membran liegt, welche die Wirkstoffabgabe in die oberste Schicht der Haut steuert.

13. Transdermales therapeutisches System nach einem oder mehreren der Ansprüche 1 bis 11, **dadurch gekennzeichnet, dass** es die Form eines Matrix-Pflasters hat, bei der Wirkstoff in einer aus einer oder mehreren Schichten bestehenden Matrix enthalten ist.

14. Transdermales therapeutisches System nach einem oder mehreren der Ansprüche 1 bis 13, **dadurch gekennzeichnet, dass** es eine äußere Deckschicht umfaßt und dass das den Wirkstoff unbrauchbar machende Agens innen an der äußeren Deckschicht fixiert ist, wobei sich das Agens bevorzugt als Lösung in einem Beutel befindet.

15. Verwendung eines transdermalen therapeutischen Systems nach einem oder mehreren der Ansprüche 1 bis 14 in der Schmerztherapie.
